# EUROPÄISCHE PATENTSCHRIFT

(11) **EP 3 247 497 B1**
(45) Veröffentlichungstag und Bekanntmachung des Hinweises auf die Patenterteilung: **01.09.2021**
(21) Anmeldenummer: 15823316.3
(22) Anmeldetag: 23.12.2015
(51) Int. Cl.: B01L 3/00, A61B 10/02, G01N 33/487

(54) **UNTERSUCHUNGSVERFAHREN, SCHEIBENFÖRMIGER PROBENTRÄGER UND VERWENDUNG EINES PROBENTRÄGERS**
ANALYSIS METHOD, DISCOID SAMPLE HOLDER AND USE OF A SAMPLE HOLDER
PROCÉDÉ D'ANALYSE, PORTE-ÉCHANTILLON DISCOÏDE ET UTILISATION D'UN PORTE-ÉCHANTILLON

(30) Priorität: 23.12.2014 DE 102014019526
(43) Veröffentlichungstag der Anmeldung: 29.11.2017
(73) Patentinhaber: Testo SE & Co. KGaA, 79853 Lenzkirch (DE)
(72) Erfinder: MUHL, Mike, 79106 Freiburg i. Brsg. (DE); BRUGGER, Simon, 79853 Lenzkirch (DE); ZUBLER, Martin, 79853 Lenzkirch (DE)
(74) Vertreter: Börjes-Pestalozza, Henrich
(86) Internationale Anmeldenummer: PCT/EP2015/002612
(87) Internationale Veröffentlichungsnummer: WO 2016/102071

(56) Entgegenhaltungen:
- EP-A1- 2 602 333
- EP-A1- 2 982 436
- EP-A2- 1 188 482
- CN-Y- 200 978 283
- US-A1- 2013 295 663
- US-A9- 2005 059 165

## Beschreibung

Die Erfindung betrifft ein Untersuchungsverfahren für eine Probe, wobei die Probe mit einem Probennahmeinstrument aufgenommen wird und wenigstens ein Verarbeitungsschritt an der Probe auf einem scheibenförmigen Probenträger durchgeführt wird.

Derartige Verfahren sind bekannt und werden beispielsweise dazu verwendet, ein Vorhandensein von ausgewählten Substanzen und/oder Mikroorganismen auf einer Oberfläche nachzuweisen und zu untersuchen. Hierzu wird mit dem Probennahmeinstrument, beispielsweise einem Tupfer, eine Probe von der Oberfläche aufgenommen und anschließend mit einem auf die jeweilige Untersuchung maßgeschneiderten Verarbeitungsschritt oder mehreren derartigen Verarbeitungsschritten untersucht.

Die Erfindung betrifft weiter einen scheibenförmigen Probenträger mit Mitteln zur Durchführung wenigstens eines Verarbeitungsschrittes an einer Probe.

Diverse Untersuchungsverfahren für Proben und Probenträger sind aus den Druckschriften EP 2 602 333 A1, CN 200 978 283 A, US 2005/059165 A9, EP 1 188 482 A2 und EP 2 982 436 A1 vorbekannt.

Der Einsatz von scheibenförmigen Probenträgern hat sich bei der Automatisierung von Verarbeitungsschritten bewährt.

Die Erfindung betrifft schließlich die Verwendung eines scheibenförmigen Probenträgers bei einem Untersuchungsverfahren.

Der Erfindung liegt die Aufgabe zu Grunde, die Handhabung in einem Untersuchungsverfahren für eine Probe zu vereinfachen.

Erfindungsgemäß sind zur Lösung dieser Aufgabe die Merkmale von Anspruch 1 vorgesehen. Insbesondere wird somit zur Lösung der genannten Aufgabe bei einem Untersuchungsverfahren der eingangs beschriebenen Art vorgeschlagen, dass das Probennahmeinstrument mit der aufgenommenen Probe in eine Aufnahme des Probenträgers eingeführt wird und dass die Probe von dem Probennahmeinstrument abgelöst und für den wenigstens einen Verarbeitungsschritt bereitgestellt wird. Von Vorteil ist dabei, dass ein Ablösen der Probe von dem Probennahmeinstrument in oder an dem Probenträger durchführbar ist. Eine Zwischenbehandlung der Probe zwischen der Aufnahme der Probe von der bereits erwähnten Oberfläche und der Durchführung des Verarbeitungsschrittes an der Probe kann somit entfallen. Die Handhabung bei dem Untersuchungsverfahren ist somit vereinfacht, insbesondere da alle Verarbeitungsschritte ab Aufnahme der Probe auf dem Probennahmeinstrument in oder an dem Probenträger ausführbar sind. Dies ermöglicht beispielsweise, das Probennahmeinstrument und den Probenträger als Wegwerfteile auszubilden, die für eine Einmalverwendung verwendbar sind. Hierdurch lassen sich Hygiene- und/oder Reinheitsanforderungen besser erfüllen.

Bei einer Ausgestaltung gemäss der Erfindung wird die Probe von dem Probennahmeinstrument durch ein Bewegen des Probenträgers abgelöst. Von Vorteil ist dabei, dass ein Einsatz von zusätzlichen Substanzen zum Ablösen der Probe von dem Probennahmeinstrument verzichtbar ist. Die Bewegung kann beispielsweise ein Schütteln oder Schwenken sein. Besonders günstig ist es dabei, wenn die Probe von dem Probennahmeinstrument durch ein Rotieren des Probenträgers abgelöst wird. Es hat sich herausgestellt, dass durch das Rotieren und die dabei entstehenden Zentrifugalkräfte ein besonders effizientes Ablösen der Probe von dem Probennahmeinstrument erreichbar ist.

Bei einer Ausgestaltung der Erfindung kann vorgesehen sein, dass die abgelöste Probe vor dem wenigstens einen Verarbeitungsschritt in einer Kammer gesammelt wird. Von Vorteil ist dabei, dass die vollständig abgelöste Probe nach Beendigung des Ablösens unmittelbar dem wenigstens einen Verarbeitungsschritt zuführbar ist. Von Vorteil ist weiter, dass der Probenträger nach dem Ablösen angehalten werden kann. Dies ist insbesondere dann günstig, wenn das Ablösen durch das bereits erwähnte Rotieren erfolgt, da dann der wenigstens eine Verarbeitungsschritt nicht notwendig unter dem Einfluss von Zentrifugalkräften ausgeführt werden muss.

Bei einer Ausgestaltung der Erfindung kann vorgesehen sein, dass während des Ablösens ein bereits abgelöster Probenteil in der Kammer mit einer Auffangflüssigkeit gemischt wird. Von Vorteil ist dabei, dass ein zu starkes Anhaften der abgelösten Probe an einer Wandung der Kammer vermeidbar oder zumindest reduzierbar ist. Von Vorteil ist weiter, dass die Probe in einer Auffangflüssigkeit für den wenigsten einen Verarbeitungsschritt bereitstellbar ist.

Bei einer Ausgestaltung der Erfindung kann vorgesehen sein, dass an die Kammer nach Ablösen der Probe ein externer Förderdruck angelegt wird, um die abgelöste Probe für den wenigstens einen Verarbeitungsschritt zu fördern. Bevorzugt wird hierzu die Bewegung des Probenträgers eingestellt oder beendet. Von Vorteil ist dabei, dass eine definierte Strömungssituation zur Durchführung des Verarbeitungsschrittes erreichbar ist.

Bei einer Ausgestaltung der Erfindung kann vorgesehen sein, dass die Aufnahme nach Einführen des Probennahmeinstruments nach außen verschlossen wird. Von Vorteil ist dabei, dass ein Herausfallen des Probennahmeinstruments beim Bewegen des Probenträgers verhinderbar ist. Von Vorteil ist dabei weiter, dass ein Ausfließen der abgelösten Probe aus der Aufnahme verhinderbar ist. Besonders günstig ist es hierbei, wenn die Aufnahme flüssigkeits- und/oder gasdicht verschlossen wird, beispielsweise mit einem Klebestreifen- und/oder mit einem Stopfen oder einem Verschlussmittel. Von Vorteil ist dabei, dass ein bereits erwähnter externer Förderdruck an die Aufnahme anlegbar ist, um die abgelöste Probe möglichst vollständig auszutragen. Alternativ oder zusätzlich kann vorgesehen sein, dass die Einführöffnung mit einem an dem Probennahmeinstrument ausgebildeten Verschlussmittel verschließbar ist und/oder verschlossen wird.

Bei einer Ausgestaltung der Erfindung kann vorgesehen sein, dass der wenigstens einen Verarbeitungsschritt ein Zytometrieverfahren ist. Von Vorteil ist dabei, dass das Vorhandensein und/oder die Anzahl von Mikroorganismen in der Probe einfach in dem Verarbeitungsschritt feststellbar ist/sind.

Bei einer Ausgestaltung der Erfindung kann vorgesehen sein, dass die abgelöste Probe vor Eintritt in eine, beispielsweise die bereits erwähnte, Kammer gefiltert wird. Von Vorteil ist dabei, dass Bestandteile des Probennahmeinstruments, die beim Ablösevorgang zusätzlich lose werden, von dem wenigstens einen Verarbeitungsschritt fernhaltbar sind.

Bei einer Ausgestaltung der Erfindung kann vorgesehen sein, dass als Probennahmeinstrument ein Tupfer verwendet wird. Somit kann die Probe besonders einfach von einer Oberfläche aufgenommen werden. Besonders günstig ist es dabei, wenn der Tupfer einen Tupferkopf aufweist, der aus von einem festen Kern abstehenden Härchen gebildet ist. Es hat sich herausgestellt, dass die Probe von derartigen Härchen besonders gut ablösbar ist. Ein Grund kann möglicherweise darin bestehen, dass die Härchen im Gegensatz zu herkömmlichen Probennahmematerialien unverflochten an dem Kern anordenbar oder angeordnet sind, sodass eine aufgenommene Probe durch Bewegungs-und/oder Schleudervorgänge besonders einfach ablösbar ist.

Bei einer Ausgestaltung der Erfindung kann vorgesehen sein, dass das Probennahmeinstrument durch die Aufnahme in einem Winkel zu einer radialen Richtung in Bezug auf ein Drehzentrum des scheibenförmigen Probenträgers positioniert wird. Die Aufnahme kann hierbei in einer durch den Probenträger vorgegebenen Ebene liegen oder auch mit dieser einen Winkel einschließen. Es ist somit vermeidbar, dass ein Koppelmittel oder eine Ankopplung des Probenträgers beim Einführen des Probennahmeinstruments behindert.

Alternativ oder zusätzlich kann bei einer Ausgestaltung der Erfindung vorgesehen sein, dass das Probennahmeinstrument durch die Aufnahme von einer Einführöffnung, insbesondere an einem äußeren Rand des scheibenförmigen Probenträgers, an einem Drehzentrum, beispielsweise dem bereits erwähnten Drehzentrum, vorbei und bis zu dem oder einem Aufnahmeende auf einer gegenüberliegenden Seite des Drehzentrums positioniert wird. Somit ist erreichbar, dass die Probe vom Probennahmeinstrument zum Aufnahmeende hin abgelöst wird, wenn der Probenträger beispielsweise zentrifugiert wird.

Bei einer Ausgestaltung der Erfindung kann vorgesehen sein, dass die Probe auf dem Probennahmeinstrument in einem saugfähigen Probennahmematerial in die Aufnahme eingeführt wird. Von Vorteil ist dabei, dass ein sicherer Transport vom Aufnahmeort zu dem Probenträger einfach ausführbar ist. Besonders günstig ist es dabei, wenn das Probennahmematerial vorbefeuchtet ist, also einen Feuchtegehalt vor Aufnahme der Probe aufweist. Somit kann auf einfache Weise erreicht werden, dass die Probe einfach von dem Probennahmematerial ablösbar ist.

Zur Lösung der genannten Aufgabe sind bei einem scheibenförmigen Probenträger die Merkmale des Anspruchs 8 erfindungsgemäß vorgesehen. Insbesondere wird somit erfindungsgemäß zur Lösung der genannten Aufgabe bei einen scheibenförmigen Probenträger der eingangs beschriebenen Art vorgeschlagen, dass eine Aufnahme für ein eine Probe tragendes Probennahmeinstrument ausgebildet ist und dass Mittel zum Ablösen der Probe von dem Probennahmeinstrument ausgebildet sind. Von Vorteil ist dabei, dass ein Ablösen der Probe von dem Probennahmeinstrument in dem scheibenförmigen Probenträger ausführbar ist. Zwischenschritte zwischen der Aufnahme der Probe von einer Oberfläche oder von einem anderem Probennahmeort einerseits und dem wenigstens einen Verarbeitungsschritt in dem Probenträger andererseits, die außerhalb des Probenträgers ablaufen müssen, sind somit verzichtbar. Dies vereinfacht die Handhabung des Probenträgers bei einem erfindungsgemäßen Untersuchungsverfahren erheblich.

Bei einer Ausgestaltung gemäss der Erfindung weisen die Mittel zum Ablösen ein Koppelmittel zum bewegungsfesten Ankoppeln des Probenträgers an einen Antrieb auf. Von Vorteil ist dabei, dass ein mechanisches Ablösen der Probe maschinell ausführbar ist, wobei zusätzliche Substanzen zum Ablösen verzichtbar sind. Besonders günstig ist es dabei, wenn das Koppelmittel zum drehfesten Ankoppeln an einen Drehantrieb ausgebildet ist. Von Vorteil ist dabei, dass Zentrifugalkräfte entwickelbar sind, welche ein besonders effizientes Ablösen der Probe von dem Probennahmeinstrument bewirken.

Bei einer Ausgestaltung der Erfindung kann vorgesehen sein, dass das Koppelmittel zum drehfesten Ankoppeln an einen Drehantrieb ausgebildet ist und dass sich die Aufnahme von einer nach außen öffnenden Einführöffnung zu einem Aufnahmeende erstreckt, wobei ein radialer Abstand der Aufnahme von einem durch das Koppelmittel definierten Drehzentrum längs der Aufnahme zwischen der Einführöffnung und dem Aufnahmeende ein Minimum annimmt. Somit ist auf einfache Weise erreichbar, dass das Aufnahmeende in Bezug auf das Drehzentrum radial weiter außen angeordnet ist als die Aufnahme im Bereich des erwähnten Minimums. Somit kann verhindert werden, dass während einer Rotation die am Aufnahmeende abgelöste Probe zurück zur Einführöffnung fließt.

Erfindungsgemäß ist vorgesehen, dass die Aufnahme für eine Einführung des Probennahmeinstruments in einer durch den Probenträger vorgegebenen Scheibenebene oder in einem spitzen Winkel hierzu ausgerichtet ist. Von Vorteil ist dabei, dass eine kompakte Außenabmessung des Probenträgers mit eingeführten Probennahmeinstruments bereitstellbar ist. Der spitze Winkel beträgt weniger als 45°. Bei einem Winkel von 0° wird das Probennahmeinstrument somit in der Scheibenebene eingeführt. Bei dieser Ausgestaltung wird somit allgemein erreicht, dass eine axiale Abmessung des Probenträgers mit eingestecktem oder eingeführtem Probennahmeinstrument klein ist, insbesondere im Vergleich zu einem Durchmesser des Probenträgers.

Bei einer Ausgestaltung der Erfindung kann vorgesehen sein, dass die Aufnahme in eine Kammer, insbesondere die bereits erwähnte Kammer, mündet. Von Vorteil ist dabei, dass die abgelöste Probe vor dem wenigsten einen Verarbeitungsschritt auffangbar und sammelbar ist. Besonders günstig ist es dabei, wenn die Kammer in einer Strömungsrichtung der abgelösten Probe hinter einem, insbesondere dem bereits erwähnten, Aufnahmeende der Aufnahme angeordnet ist. Von Vorteil ist dabei, dass ein Aufsammeln der Probe in der Kammer einfach durchführbar ist. Insbesondere bei einem Koppelmittel, das zu einem drehfesten Koppeln an einen Drehantrieb ausgebildet ist, ist es günstig, wenn die Kammer radial in Bezug auf ein Drehzentrum des Koppelmittels außerhalb des Aufnahmeendes angeordnet ist. Von Vorteil ist dabei, dass die auftretenden Zentrifugalkräfte eine Strömungsrichtung der abgelösten Probe auf die Kammer zu bewirken.

Bei einer Ausgestaltung der Erfindung kann vorgesehen sein, dass in einer, beispielsweise in der bereits erwähnten, Strömungsrichtung hinter der Aufnahme ein Filter angeordnet ist. Von Vorteil ist dabei, dass Bruchteile oder sonstige Bestandteile des Probennahmeinstruments, die beim Ablösevorgang lose geworden sind, von dem wenigstens einen Verarbeitungsschritt fernhaltbar sind und/oder ferngehalten werden.

Bei einer Ausgestaltung der Erfindung kann vorgesehen sein, dass in der Aufnahme ein Anlagevorsprung ausgebildet ist, an welchem ein eingeführtes Probennahmeinstrument punkt- oder linienförmig anliegt und/oder gehalten ist. Von Vorteil ist bei, dass ein flächiges Anliegen des Probennahmeinstruments vermeidbar ist, sodass ein Ablösen der Probe von den Probennahmeinstrument einfach erreichbar ist.

Bei einer Ausgestaltung der Erfindung kann vorgesehen sein, dass ein Zytometerkanal ausgebildet ist, welcher an die Aufnahme angeschlossen ist. Die Mittel zur Durchführung des wenigstens eines Verfahrensschrittes umfassen daher in diesem Fall zumindest den Zytometerkanal. Von Vorteil ist dabei, dass als wenigstens ein Verarbeitungsschritt ein Zytometrieverfahren ausführbar ist.

Bei einer Ausgestaltung der Erfindung kann vorgesehen sein, dass ein mit einer, insbesondere der bereits erwähnten, Kammer verbundenes Flüssigkeitsreservoir ausgebildet und mit einer Auffangflüssigkeit befüllt ist. Von Vorteil ist dabei, dass ein Mischen der abgelösten Probe mit der Auffangflüssigkeit in den Probenträger ausführbar ist.

Bei einer Ausgestaltung der Erfindung kann vorgesehen sein, dass ein Druckanschluss für einen externen Förderdruck ausgebildet ist. Von Vorteil ist dabei, dass ein Fördern der abgelösten Probe zu dem wenigstens einen Verarbeitungsschritt auch bei stillstehendem Probenträger einfach ausführbar ist.

Bei einer Ausgestaltung der Erfindung kann vorgesehen sein, dass das Probennahmeinstrument ein saugfähiges Probennahmematerial aufweist. Somit bildet der Probenträger mit dem Probennahmematerial ein Probenträger-Set. Die Verwendung eines saugfähigen Probennahmematerials ist besonders günstig, wenn eine Probe von einer Oberfläche aufnehmbar sein und/oder aufgenommen werden soll. Besonders günstig ist es hierbei, wenn das Probennahmematerial vorbefeuchtet ist. Bei dem erfindungsgemäßen Probenträger-Set kann das Probennahmeinstrument, beispielsweise ein Tupfer mit dem vorbefeuchteten saugfähigem Probennahmematerial, separat von den Probenträger verpackt und bereitgehalten sein.

Bei einer Ausgestaltung der Erfindung kann vorgesehen sein, dass die Kammer in einer Strömungsrichtung der abgelösten Probe zulaufend ausgebildet ist. Von Vorteil ist dabei, dass ein Sammeln der abgelösten Probe einfach ausführbar ist. Hierbei kann vorgesehen sein, dass die Strömungsrichtung näherungsweise oder genau radial in Bezug auf ein Drehzentrum des Probenträgers ausgerichtet ist. Dies kann beispielsweise dann gegeben sein, wenn der Probenträger zum Ablösen der Probe um das Drehzentrum rotiert wird, sodass die Strömungsrichtung aus der Wirkung einer Zentrifugalkraft entsteht.

Bei einer Ausgestaltung der Erfindung kann vorgesehen sein, dass an einem von der Aufnahme in einer Strömungsrichtung beispielsweise der bereits erwähnten Strömungsrichtung, der abgelösten Probe abgewandten Kammerende einen Auslass aufweist. Von Vorteil ist dabei, dass ein direktes Weiterleiten der Probe zu den Mitteln zur Durchführung wenigstens eines Verarbeitungsschrittes ausführbar ist. Für den Fall, dass der Probenträger während des Ablösevorgangs rotiert werden soll, ist es günstig, wenn der Auslass an der Kammer radial außen in Bezug auf ein Drehzentrum des Probenträgers angeordnet ist.

Bei einer Ausgestaltung der Erfindung kann vorgesehen sein, dass die Aufnahme eine Verlaufsrichtung hat, welche einen Winkel einschließt mit einer radialen Richtung in Bezug auf ein Drehzentrum des scheibenförmigen Probenträgers. Somit ist die Aufnahme nahe an ein Drehzentrum heranführbar, ohne dass eine Einführbarkeit des Probennahmeinstruments verringert wird.

Bei einer Ausgestaltung der Erfindung kann vorgesehen sein, dass die Aufnahme von einer Einführöffnung an einem Drehzentrum, beispielsweise dem bereits erwähnten Drehzentrum, vorbei verläuft und auf einer gegenüberliegenden Seite des Drehzentrums endet. Von Vorteil ist dabei, dass ein Rückfluss der abgelösten Probe entlang des Probennahmeinstruments vermeidbar ist. Besonders günstig ist es, wenn die Einführöffnung an einem äußeren Rand des scheibenförmigen Probenträgers liegt. Somit ist eine große Länge der Aufnahme bereitstellbar.

Bei einer Ausgestaltung der Erfindung von möglicherweise eigenständiger erfinderischer Qualität kann vorgesehen sein, dass der scheibenförmige Probenträger aus wenigstens zwei Segmenten zusammengesetzt ist oder wird, wobei jedes Segment als erfindungsgemäßer scheibenförmiger Probenträger, insbesondere wie zuvor beschrieben und/oder nach einem der auf einen Probenträger gerichteten Ansprüche, ausgebildet ist. Somit lassen sich die zu untersuchenden Proben getrennt mit in die einzelnen Segmente aufnehmen und anschließend in dem zusammengesetzten Probenträger gemeinsam untersuchen. Besonders günstig ist es, wenn die Segmente jeweils paarweise entlang einer Trennlinie voneinander trennbar sind und eine Einführöffnung wenigstens eines Segments auf der zugehörigen Trennlinie liegt. Von Vorteil ist dabei, dass die Einführöffnung bei Zusammenlegen der Segmente verschließbar ist.

Zur Lösung der genannten Aufgabe ist erfindungsgemäß schließlich die Verwendung eines erfindungsgemäßen, scheibenförmigen Probenträgers nach einen der Ansprüche 8-16, bei einem erfindungsgemäßen Untersuchungsverfahren nach einem der Ansprüche 1-7 vorgesehen.

Hierbei kann vorgesehen sein, dass die Probe mit dem Probennahmeinstrument aufgenommen wird, dass das Probennahmeinstrument in den Probenträger eingeführt wird, dass der Probenträger mit dem eingeführten Probennahmeinstrument in ein Untersuchungsgerät eingesetzt wird und dass in dem Untersuchungsgerät die verbleibenden Schritte des erfindungsgemäßen Untersuchungsverfahren an dem Probenträger ausgeführt werden. Insgesamt kann vorgesehen sein, dass die Probe nach dem wenigsten einen Verarbeitungsschritt oder nach weiteren Verarbeitungsschritten in einer Speicherkammer des Probenträgers gesammelt wird. Somit kann der Probenträger mit der verwendeten Probe gemeinsam einer Endlagerung zugeführt werden.

Die Erfindung wird nun anhand von Ausführungsbeispielen näher erläutert, ist jedoch nicht auf diese Ausführungsbeispiele beschränkt. Weitere Ausführungsbeispiele ergeben sich durch Kombination der Merkmale einzelner oder mehrerer Schutzansprüche untereinander.

Es zeigt:
- Fig. 1: einen erfindungsgemäßen scheibenförmigen Probenträger in stark schematisierter Darstellung,
- Fig. 2: ein Probennahmeinstrument zur Verwendung in einem erfindungsgemäßen Untersuchungsverfahren
- Fig. 3: die Verwendung eines Probennahmeinstruments an einem Probenträger in stark vereinfachter Darstellung,
- Fig. 4: den Probenträger, gemäß Figur 1, mit eingesetztem Probennahmeinstrument,
- Fig. 5: einen weiteren erfindungsgemäßen Probenträger, der aus fünf scheibenförmigen Probenträgern in Form von Segmenten zusammengesetzt ist,
- Fig. 6: ein Segment aus Fig. 5,
- Fig. 7: das Einführen eines Probennahmeinstruments in das Segment aus Fig. 6,
- Fig. 8: eine Schnittdarstellung durch das Segment aus Fig. 6,
- Fig. 9: einen weiteren erfindungsgemäßen Probenträger, der aus fünf scheibenförmigen Probenträgern in Form von Segmenten zusammengesetzt ist, mit radial vom Drehzentrum weg verlaufenden Aufnahmen,
- Fig. 10: ein Segment aus Fig. 9,
- Fig. 11: das Einführen eines Probennahmeinstruments in das Segment aus Fig. 10,
- Fig. 12: einen weiteren erfindungsgemäßen Probenträger, der aus fünf scheibenförmigen Probenträgern in Form von Segmenten zusammengesetzt ist, mit schräg in einem Winkel zur Radialrichtung verlaufenden Aufnahmen,
- Fig. 13: ein Segment aus Fig. 12 und
- Fig. 14: das Einführen eines Probennahmeinstruments in das Segment aus Fig. 13.

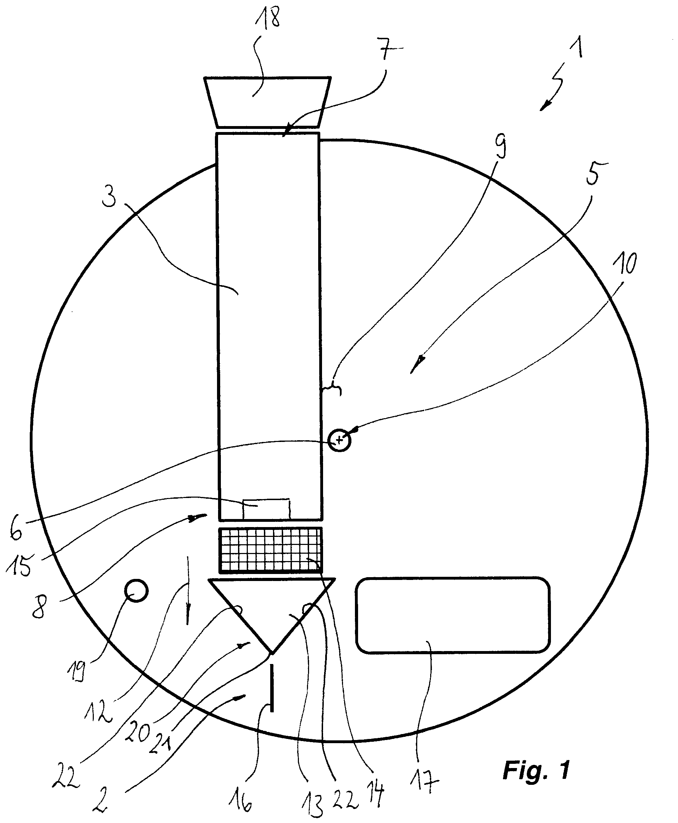

Ein in Figur 1 im Ganzen mit 1 bezeichneter scheibenförmiger Probenträger weist noch näher zu beschreibende Mittel 2 zur Durchführung wenigstens eines Verarbeitungsschrittes an einer Probe auf.

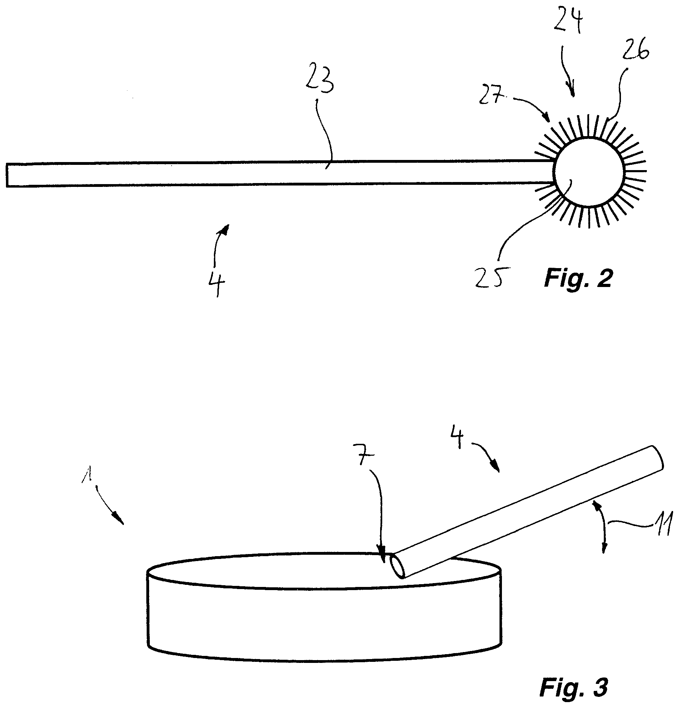

Der Probenträger 1 weist eine Aufnahme 3 auf, in welche die bereits erwähnte Probe für den wenigstens einen Verarbeitungsschritt auf einem noch genauer zu beschreibenden und in Figur 2 näher dargestellten Probennahmeinstrument 4 einführbar ist.

Bei der Verwendung des Probenträgers 1 bei dem erfindungsgemäßen Untersuchungsverfahren wird daher die Probe mit dem Probennahmeinstrument 4 aufgenommen und dann mit dem Probennahmeinstrument 4 in die Aufnahme 3 verbracht.

Der Probenträger 1 weist Mittel 5 zum Ablösen der Probe von dem eingesetzten Probennahmeinstrument 4 auf. Diese Mittel 5 umfassen insbesondere ein Koppelmittel 6, mit welchem der Probenträger 1 drehfest an einen nicht weiter dargestellten Drehantrieb, beispielsweise des bereits erwähnten Untersuchungsgeräts, koppelbar ist.

Mit dem Drehantrieb wird der Probenträger 1 rotiert, um über die resultierende Zentrifugalkraft die Probe von dem Probennahmeinstrument 4 in der Aufnahme 3 abzulösen.

Bei weiteren Ausführungsbeispielen ist statt des Drehantriebs ein allgemeiner Antrieb vorgesehen, an dem der Probenträger 1 über das Koppelmittel 6 bewegungsfest ankoppelbar ist, um - beispielsweise durch Schütteln - die Probe von dem Probennahmeinstrument 4 abzulösen.

Die Aufnahme 3 in Figur 1 weist eine nach außen offene Einführöffnung 7 auf, durch welche das Probennahmeinstrument 4 einführbar ist.

Die Aufnahme 3 weist weiter ein Aufnahmeende 8 auf, an welchem das Ablösen der Probe von dem Probennahmeinstrument 4 stattfindet.

Zwischen der Einführöffnung 7 und dem Aufnahmeende 8 weist die Aufnahme 3 einen geradlinigen Verlauf auf.

Die Anordnung der Aufnahme 3 ist hierbei derart, dass ein variabler, radialer Abstand 9 der Aufnahme 3, beispielsweise jeweils gemessen von einem variablen Punkt auf einer Mittellinie der Aufnahme 3, von einem Drehzentrum 10 des Koppelmittels 6 weder an der Einführöffnung 7 noch an dem Aufnahmeende 8, sondern zwischen dem Aufnahmeende 8 und der Einführendung 7 ein Minimum aufweist. Auf diese Weise wird erreicht, dass das Aufnahmeende 8 radial in Bezug auf das Drehzentrum 10 außerhalb eines durch dieses Minimum gegebenen Abschnittes der Aufnahme 3 angeordnet ist. Somit fließt eine von dem eingesetzten Probennahmeinstrument 4 abgelöste Probe nicht zur Einführöffnung 7, sondern zu dem Aufnahmeende 8. Somit läuft die Aufnahme 3 von der Einführöffnung 7 an dem Drehzentrum 10 vorbei, sodass die Einführöffnung 7 auf einer Seite des Drehzentrums 10 und das Aufnahmeende 8 auf einer gegenüberliegenden Seite des Drehzentrums 10 liegen.

In Figur 3 ist das Einführen des Probennahmeinstruments 4 in die Aufnahme 3 durch die Einführöffnung 7 gezeigt. Es ist ersichtlich, dass das Probennahmeinstrument 4 in einem spitzen Winkel 11, der beispielsweise kleiner als 45° sein kann und in Figur 1 0° beträgt, in dem Probenträger 1 eingeführt wird. Die Aufnahme 3 verläuft hierbei beispielweise von der Einführöffnung 7 bis auf die gegenüberliegende Seite des Probenträgers 1. Ein Tupferkopf 24 (vgl. Fig. 2) und das von diesem entfernte Ende des Tupferstiels eines Probennahmeinstruments 4 sind somit in der Aufnahme 3 auf einander gegenüber liegenden Seiten angeordnet. Das eingeführte Probennahmeinstrument 4 verläuft somit am Drehzentrum 10 vorbei.

Bei Rotation des Probenträgers 1 um das Drehzentrum 10 mit eingeführten Probennahmeinstrument 4 ist durch die Zentrifugalkräfte eine Strömungsrichtung 12 vorgegeben.

In Strömungsrichtung 12 hinter dem Aufnahmeende 8 der Aufnahme 3 ist eine Kammer 13 angeordnet, in welcher die Aufnahme 3 mündet.

Die Kammer 13 dient zum Auffangen der abgelösten Probe während der Rotation des Probenträgers 1.

Hinter dem Aufnahmeende 8 ist in Strömungsrichtung 12 zwischen der Aufnahme 3 und der Kammer 13 ein Filter 14 ausgebildet, mit welchem nicht flüssige Bestandteile des Probennahmeinstruments 4, also beispielsweise Bruchstücke oder Fasern, auffangbar und gegenüber der Kammer 13 zurückhaltbar sind.

In der Aufnahme 3 ist am Aufnahmeende 8 ein Anlagevorsprung 15 ausgebildet, an welchem das Probennahmeinstrument 4 in der Aufnahme 3 anliegen kann.

Hierzu ist der nur symbolisch dargestellte Anlagevorsprung 15 so geformt, dass das Probennahmeinstrument 4 punkt- oder linienförmig anliegt und gehalten ist. Beispielsweise kann der Anlagevorsprung 15 hierzu eine Spitze oder Grate aufweisen.

Bei den gezeigten Ausführungsbeispielen- weisen die Mittel 2 zur Durchführung des wenigstens einen Verarbeitungsschnittes einen Zytometerkanal 16 auf, mit welchem in an sich bekannter Weise ein optisches Zytometrieverfahren ausführbar ist.

Hierzu werden an den Probenträger 1 eine externe Lichtquelle und ein externer Lichtdetektor des Untersuchungsgeräts angeordnet, die an sich bekannt und hier nicht weiter dargestellt sind.

Der Probenträger 1 weist ein Flüssigkeitsreservoir 17 auf in welchem eine Auffangflüssigkeit vorgehalten ist.

Das Flüssigkeitsreservoir 17 ist hierbei zu der Kammer 13 geöffnet oder öffenbar, sodass die Auffangflüssigkeit aus dem Flüssigkeitsreservoir 17 in die Kammer 13 verbringbar ist. Die befüllte Kammer 13 ist somit vorbereitet zum Auffangen der abgelösten Probe von dem Probennahmeinstrument 4. In der Kammer 13 wird somit die aufgefangene Probe mit der Auffangflüssigkeit aus der Flüssigkeitsreservoir 17 gemischt. Dies kann während des Ablösens, also wenn erst ein Probenteil der Probe in die Kammer 13 gelangt ist, oder nach dem Ablösen der Probe erfolgen.

Um zu verhindern, dass während der Rotation des Probenträgers 1 das Probennahmeinstrument 4 und/oder die abgelöste Probe aus der Aufnahme 3 durch die Einführöffnung 7 austritt/austreten, wird die Einführöffnung 7 durch einen Stopfen 18 verschlossen. Statt des Stopfens 18 ist auch ein Klebestreifen oder allgemein ein Verschlussmittel verwendbar. Hier kann der in Figur 1 kreisrund gezeichnete Probenträger 1 eine Abplattung beispielsweise durch eine Sekante aufweisen, an welcher die Einführöffnung 7 ausgebildet ist und welche mit einem flächigen Klebestreifen abklebbar ist.

Alternativ oder zusätzlich kann bei einem weiteren Ausführungsbeispiel das Probennahmeinstrument 4 mit einem Verschlussmittel zum Verschluss der Einführöffnung 7 versehen sein.

An dem Probenträger 1 ist ein Druckanschluss 19 für einen externen Förderdruck ausgebildet, der mit der Kammer 13 - beispielsweise über die Aufnahme 3 - verbunden ist. Somit kann eine in der Kammer 13, aufgefangene Probe zu dem Zytometerkanal 16 zu fördern.

In Figur 1 ist ersichtlich, dass die Kammer 13 in der Strömungsrichtung 12 vorzugsweise radial nach außen zulaufend ausgebildet ist.

An einem Kammerende 20, welches auf einer von der Aufnahme 3 abgewandten Seite der Kammer 13 befindlich ist, ist ein Auslass 21 ausgebildet, welcher mit dem Zytometerkanal 16 oder allgemein mit den Mitteln 2 zur Durchführung eines Verarbeitungsschrittes verbunden ist.

Durch die nach außen zulaufende Form der Kammer 13 sind somit Wandungen 22 der Kammer 13 gebildet, welche die aufgefangene Probe dem Verarbeitungsschritt zuführen.

In Figur 2 ist ein Probennahmeinstrument 4 in Form eines Tupfers 23 gezeigt, der einen Tupferkopf 24 aufweist.

Der Tupferkopf 24 trägt ein saugfähiges Probennahmematerial 27, das mit der aufzunehmenden Probe in Kontakt gebracht wird.

Der Tupferkopf 24 weist hierbei einen festen Kern 25 und von diesem festen Kern 25 abstehende Härchen 26 auf.

Die Härchen 26 bilden das Probennahmematerial 27 und stehen hierbei nach außen von dem Kern 25 ab. Sie sind nicht miteinander verwebt oder verflochten.

Somit kann die aufgenommene Probe durch Schütteln oder durch eine Zentrifugalkraft einfach von den Härchen 26 gelöst werden, sobald das Probennahmeinstrument 4 in der Aufnahme 3 befindlich ist.

Die Verwendung des Probennahmeinstruments 4 gemäß Figur 2 bei dem Probenträger 1 erfolgt erfindungsgemäß wie folgt:
Zunächst wird die Probe mit dem Probennahmeinstrument 4 von einer Oberfläche aufgenommen und mit dem Probennahmeinstrument 4 in die Aufnahme 3 eingeführt.

Sodann wird der Probenträger 1 über das Koppelmittel 6 mit einem Drehantrieb gekoppelt, beispielsweise durch Einsetzen des Probenträgers 1 in ein entsprechendes Untersuchungsgerät.

Nun wird die Aufnahme 3 nach außen an der Einführöffnung 7 durch einen Stopfen 18 oder durch ein anderes Verschlussmittel gas- und flüssigkeitsdicht verschlossen.

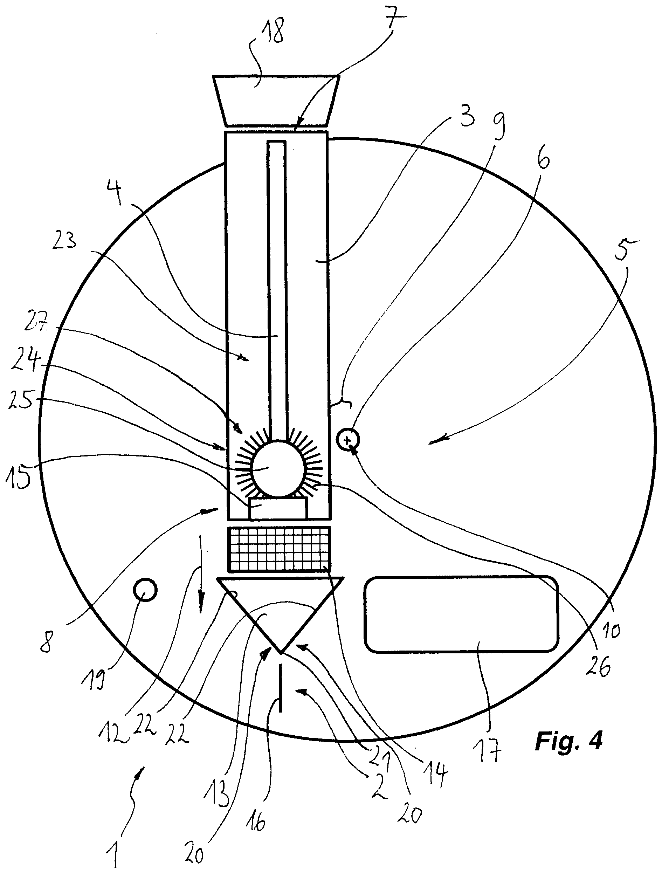

Figur 4 zeigt die Situation nach Verschluss der Einführöffnung 7 und vor Beginn des Rotierens.

In einem nächsten Schritt wird der Probenträger 1 in Rotation versetzt, sodass die Zentrifugalkraft die Probe aus dem Probennahmematerial 27, von dem Probennahmeinstrument 4 und insbesondere dem Tupferkopf 24 ablöst.

Diese Probe wird an dem Aufnahmeende 8 durch einen Filter 14 der Kammer 13 zugeführt, wo sie gesammelt wird. In der Kammer 13 wird die aufgefangene Probe mit einer Auffangflüssigkeit auf dem Flüssigkeitsreservoir 17 vermischt.

Anschließend wird der Probenträger 1 angehalten und es wird an dem Druckanschluss 19 ein externer Förderdruck angelegt.

Dieser externe Förderdruck wird über die Aufnahme 3 an die Kammer 13 angelegt, um die aufgefangene Probe den Mitteln 2 zur Durchführung des wenigstens einen Verarbeitungsschrittes zuzuführen.

Im Ausführungsbeispiel wird hierbei die Probe durch einen Zytometerkanal 16 gefördert, um ein Zytometrieverfahren auszuführen.

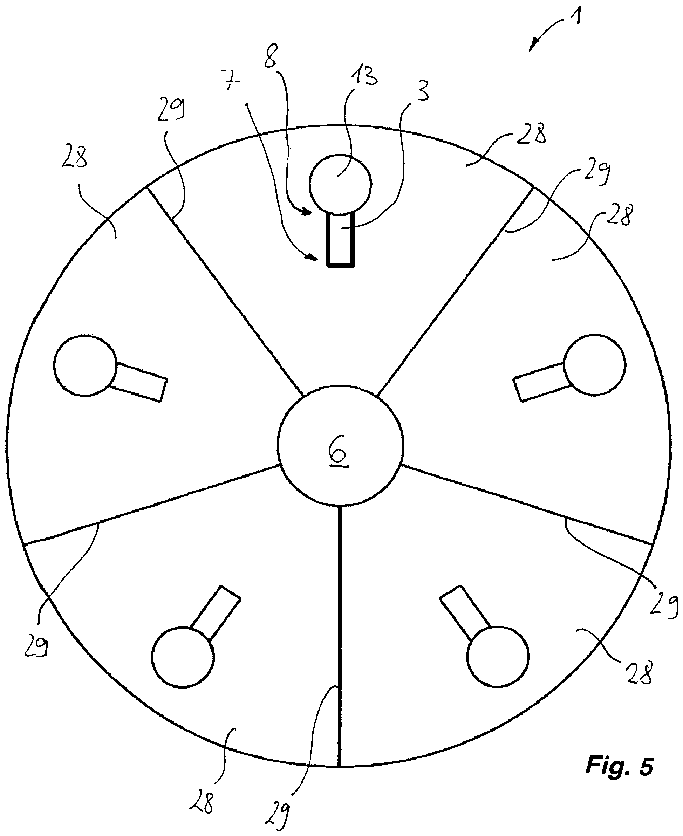

Fig. 5 zeigt einen weiteren erfindungsgemäßen Probenträger 1, der aus Segmenten 28 besteht, die an Trennlinien 29 zusammengesetzt sind. Funktionell oder konstruktiv zu den vorangegangenen Ausführungsbeispielen identische oder gleichartige Bauteile und Funktionseinheiten sind mit denselben Bezugszeichen bezeichnet und nicht gesondert beschrieben. Die Ausführungen zu den Fig. 1 bis 4 gelten daher zu Fig. 5 bis 8 entsprechend.

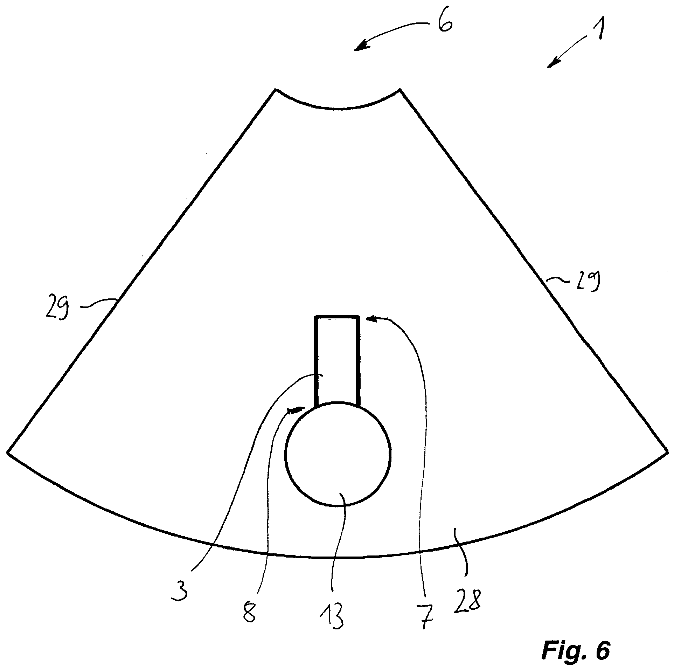

Jedes Segment 28 bildet für sich genommen einen erfindungsgemäßen Probenträger 1, der in Fig. 6 bis 8 abgebildet und jeweils so ausgebildet ist, dass er mit weiteren erfindungsgemäßen Probenträgern 1 (Segmente 28) zu einer Kreisscheibe gemäß Fig. 5 zusammensetzbar ist. Insbesondere ist jedes Segment mit vorzugsweise radial verlaufenden Trennlinien 29 ausgerüstet, um das Zusammenlegen zu ermöglichen.

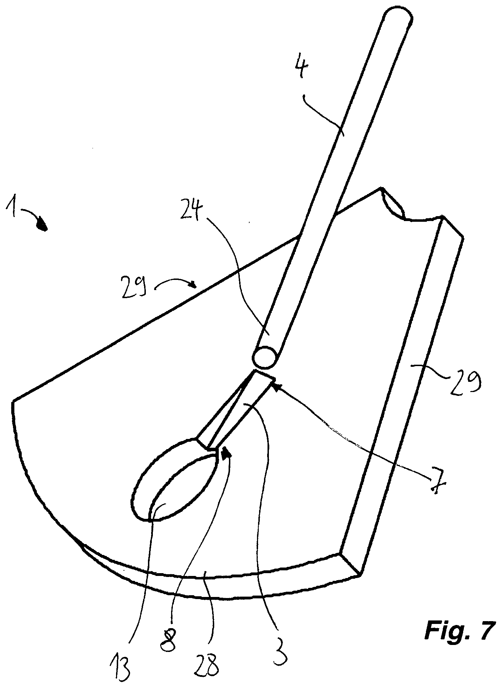

Die Aufnahmen 3 sind jeweils radial ausgerichtet und ermöglichen ein Einführen des Probennahmeinstruments 4 von außerhalb einer durch den Probenträger 1 definierten Ebene, vgl. Fig. 7.

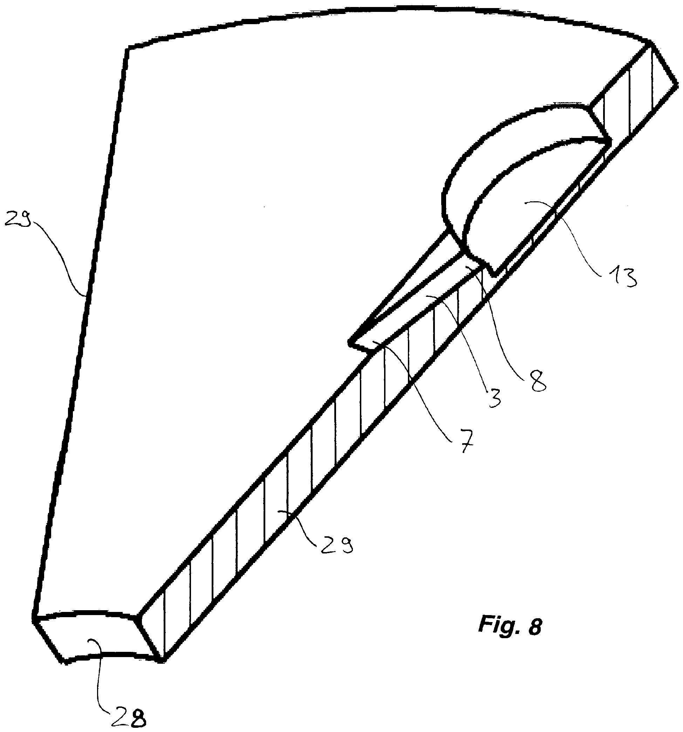

Fig. 8 zeigt, dass die Verlaufsrichtung der Aufnahme 3 mit der Scheibenebene einen Winkel einschließt.

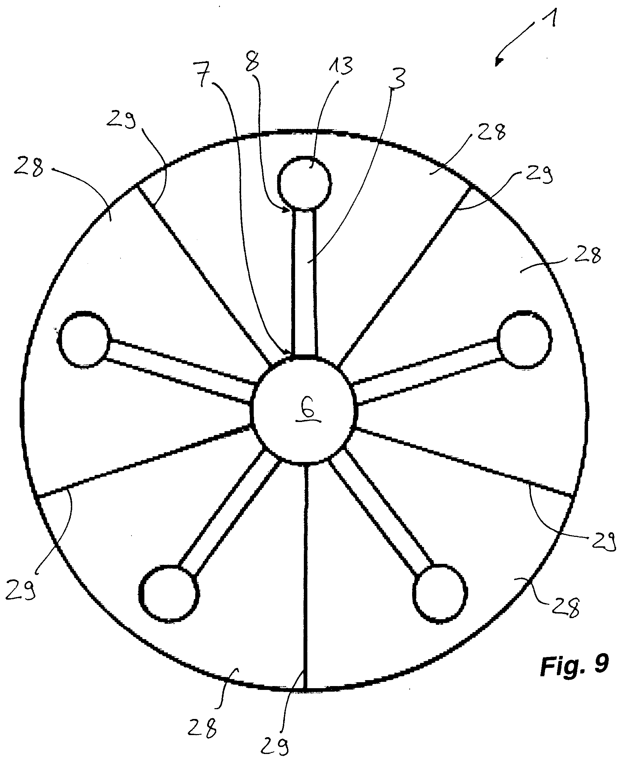

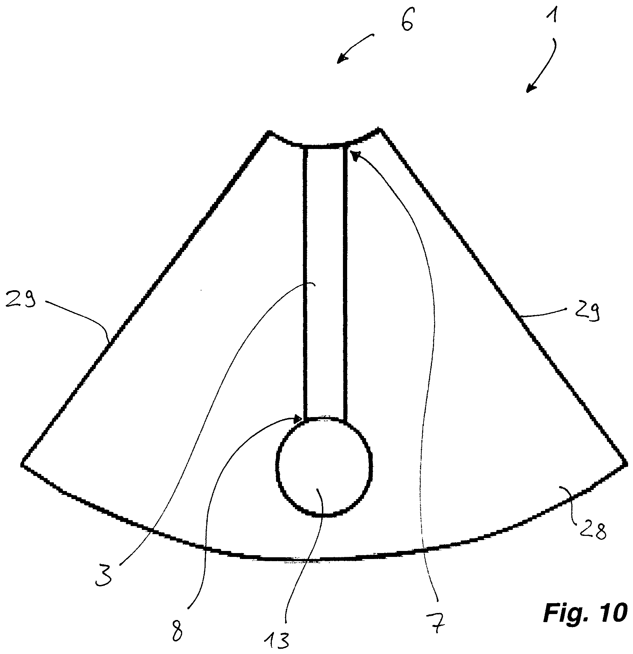

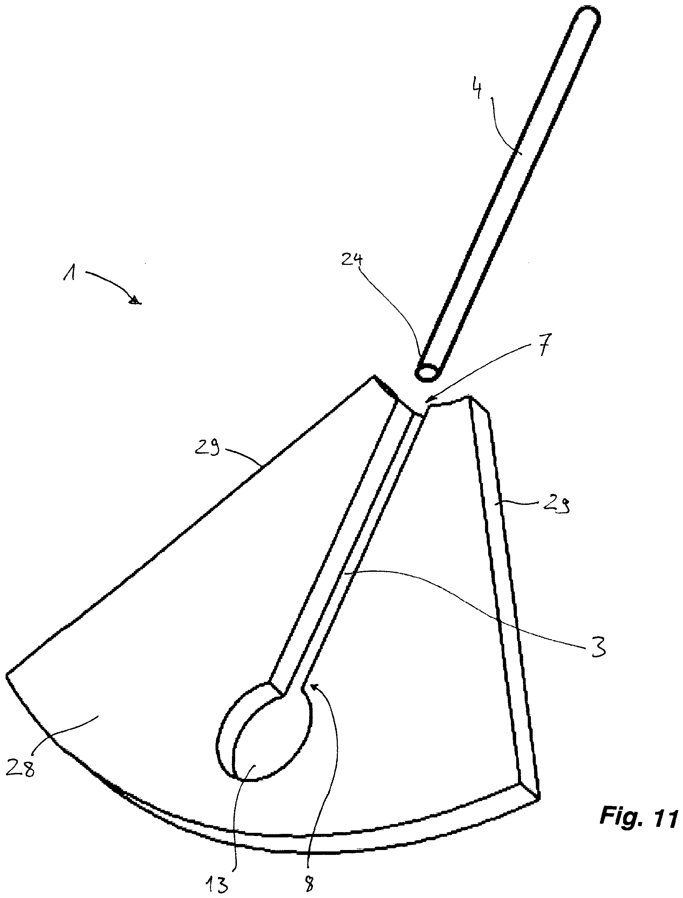

Fig. 9 zeigt einen weiteren erfindungsgemäßen Probenträger 1, der aus Segmenten 28 besteht, die an Trennlinien 29 zusammengesetzt sind. Funktionell oder konstruktiv zu den vorangegangenen Ausführungsbeispielen identische oder gleichartige Bauteile und Funktionseinheiten sind mit denselben Bezugszeichen bezeichnet und nicht gesondert beschrieben. Die Ausführungen zu den Fig. 1 bis 8 gelten daher zu Fig. 9 bis 11 entsprechend.

Die Aufnahmen 3 sind jeweils bis zum Koppelmittel 6 geführt. Ein Einführen des Probennahmeinstruments ist somit in der Scheibenebene des Probenträgers 1 möglich, bevor die Segmente 28 zusammengesetzt werden. Der zusammengesetzte Probenträger 1 in Fig. 9 hat somit eine sternförmige Anordnung von Aufnahmen 3.

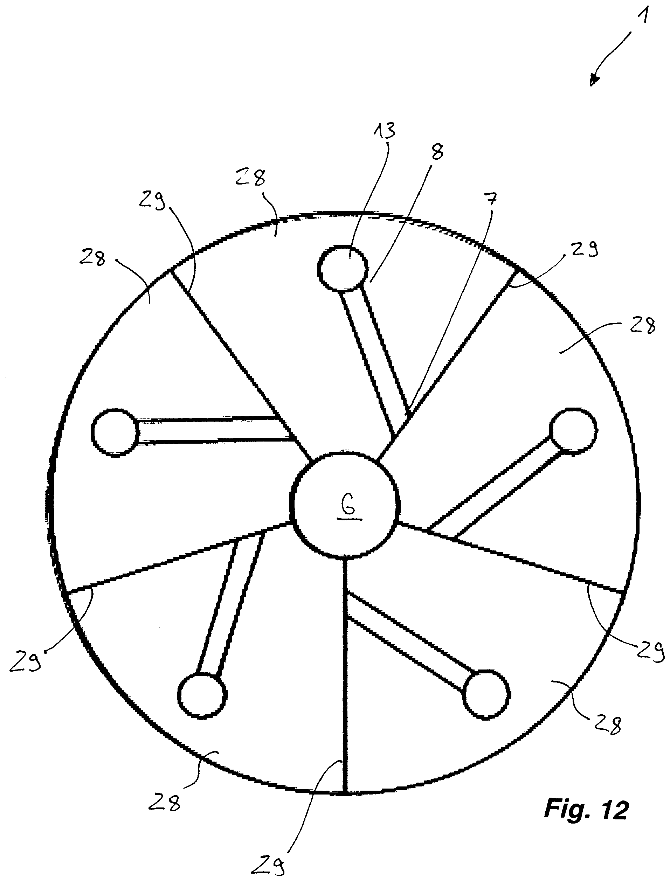

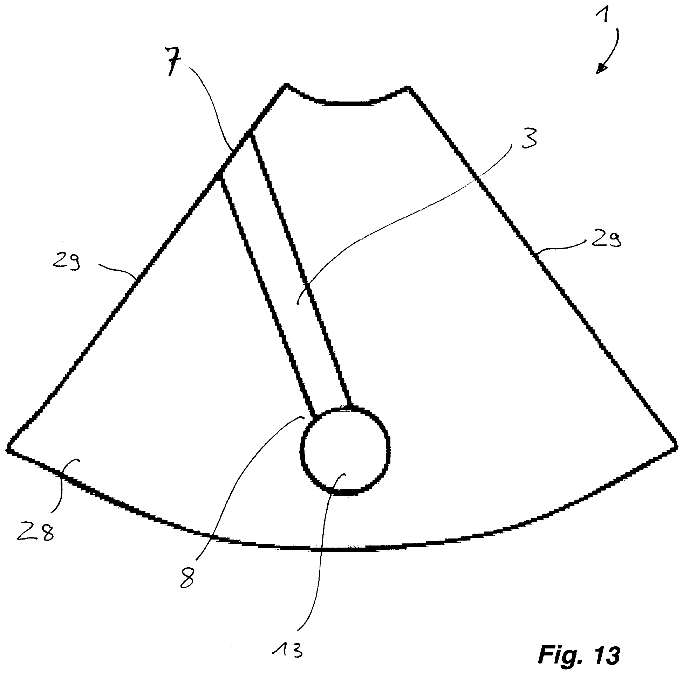

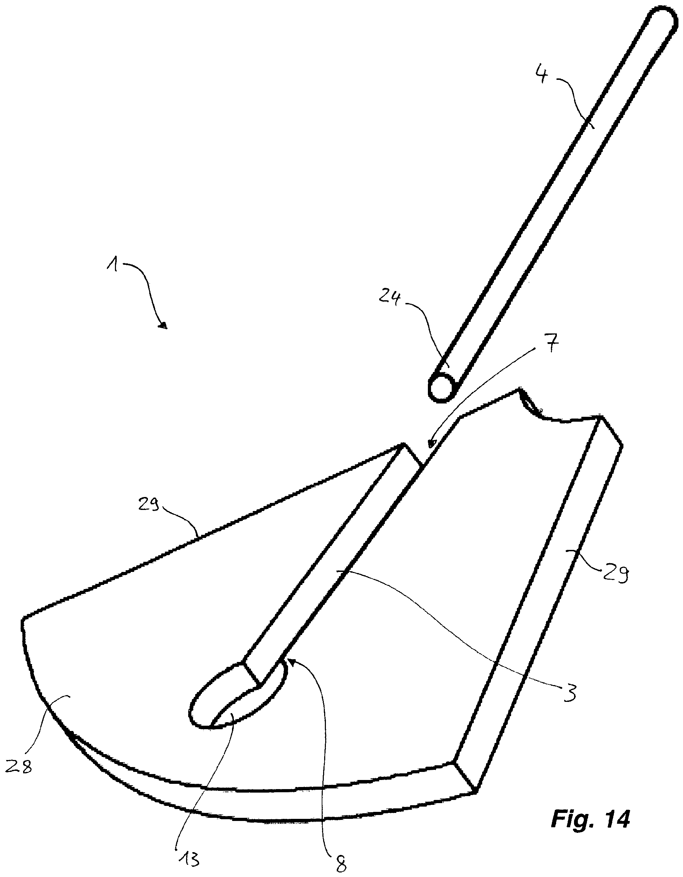

Fig. 12 zeigt einen weiteren erfindungsgemäßen Probenträger 1, der aus Segmenten 28 besteht, die an Trennlinien 29 zusammengesetzt sind. Funktionell oder konstruktiv zu den vorangegangenen Ausführungsbeispielen identische oder gleichartige Bauteile und Funktionseinheiten sind mit denselben Bezugszeichen bezeichnet und nicht gesondert beschrieben. Die Ausführungen zu den Fig. 1 bis 11 gelten daher zu Fig. 12 bis 14 entsprechend.

Die Aufnahmen 3 verlaufen hierbei jeweils von der Trennlinie 29 zu einem Aufnahmeende 8, wobei die Verlaufsrichtung der Aufnahmen 3 jeweils einen Winkel mit der auf das Drehzentrum zeigenden Radialrichtung einschließen. Die Einführöffnungen 7 sind bei zusammengesetztem Probenträger 1 durch das benachbarte Segment 28 verschlossen.

Bei den gezeigten Ausführungsbeispielen gemäß Fig. 5 bis 14 sind die Segmente 28 eines Probenträgers 1 jeweils identisch ausgebildet. Es sind jedoch auch unterschiedliche Segmente 28 kombinierbar, oder es können abweichend vom Gezeigten gestaltete Segmente zu weniger als fünf oder mehr als fünf Segmente aufweisenden Probenträgern zusammengesetzt werden.

Bei dem scheibenförmigen Probenträger 1, auf dem Mittel 2 zur Durchführung wenigstens eines Verarbeitungsschrittes ausgebildet sind, wird vorgeschlagen, eine Aufnahme 3, in welcher ein Probennahmeinstrument 4 einführbar ist, und Mittel 5 zum Ablösen einer Probe von dem in der Aufnahme 3 angeordneten Probennahmeinstrument 4 auszubilden.

## Patentansprüche

1. **Untersuchungsverfahren für eine Probe**, wobei die Probe mit einem Probennahmeinstrument (4) aufgenommen wird und wenigstens ein Verarbeitungsschritt an der Probe auf einem scheibenförmigen Probenträger (1) durchgeführt wird, wobei das Probennahmeinstrument (4) mit der aufgenommenen Probe in eine Aufnahme (3) des Probenträgers (1) eingeführt wird, wobei die Probe von dem Probennahmeinstrument (4) abgelöst und für den wenigstens einen Verarbeitungsschritt bereitgestellt wird, wobei die Probe von dem Probennahmeinstrument (4) durch ein Bewegen des Probenträgers (1) abgelöst wird, **dadurch gekennzeichnet, dass** die Aufnahme (3) für ein Einführen des Probennahmeinstruments (4) in einer durch den Probenträger (1) vorgegebenen Scheibenebene oder in einem Winkel (11) von weniger als 45° hierzu ausgerichtet ist.

2. Untersuchungsverfahren nach Anspruch 1, **dadurch gekennzeichnet, dass** die Probe von dem Probennahmeinstrument (4) durch ein Rotieren des Probenträgers (1) abgelöst wird.

3. Untersuchungsverfahren nach Anspruch 1 oder 2, **dadurch gekennzeichnet, dass** die abgelöste Probe vor dem wenigstens einen Verarbeitungsschritt in einer Kammer (13) gesammelt wird, insbesondere wobei während des Ablösens ein bereits abgelöster Probenteil in der Kammer (13) mit einer Auffangflüssigkeit gemischt wird.

4. Untersuchungsverfahren nach einem der Ansprüche 1 bis 3, **dadurch gekennzeichnet, dass** an die Kammer (13) nach Ablösen der Probe ein externer Förderdruck angelegt wird, um die abgelöste Probe für den wenigstens einen Verarbeitungsschritt zu fördern, und/oder dass die Aufnahme (3) nach Einführen des Probennahmeinstruments (4) nach außen vorzugsweise flüssigkeits- und/oder gasdicht verschlossen wird, insbesondere mit einem Klebestreifen und/oder einem Stopfen (18) und/oder mit einem an dem Probennahmeinstrument (4) ausgebildeten Verschlussmittel.

5. Untersuchungsverfahren nach einem der Ansprüche 1 bis 4, **dadurch gekennzeichnet, dass** der wenigstens eine Verarbeitungsschritt ein Zytometrieverfahren ist und/oder dass die abgelöste Probe vor Eintritt in die oder eine Kammer (13) gefiltert wird.

6. Untersuchungsverfahren nach einem der Ansprüche 1 bis 5, **dadurch gekennzeichnet, dass** als Probennahmeinstrument (4) ein Tupfer (23), insbesondere ein Tupfer (23), der einen aus von einem festen Kern (25) abstehenden Härchen (26) gebildeten Tupferkopf (24) aufweist, verwendet wird und/oder dass die Probe auf dem Probennahmeinstrument (4) in einem saugfähigen, vorzugsweise vorbefeuchteten Probennahmematerial (27) in die Aufnahme (3) eingeführt wird.

7. Untersuchungsverfahren nach einem der Ansprüche 1 bis 6, **dadurch gekennzeichnet, dass** das Probennahmeinstrument (4) durch die Aufnahme (3) in einem Winkel zu einer radialen Richtung in Bezug auf ein Drehzentrum (10) des scheibenförmigen Probenträgers (1) positioniert wird und/oder dass das Probennahmeinstrument (4) durch die Aufnahme (3) von einer Einführöffnung (7), insbesondere an einem äußeren Rand des scheibenförmigen Probenträgers (1), an dem oder einem Drehzentrum (10) vorbei und bis zu dem oder einem Aufnahmeende (8) auf einer gegenüberliegenden Seite des Drehzentrums (10) positioniert wird.

8. **Scheibenförmiger Probenträger (1)** mit Mitteln (2) zur Durchführung wenigstens eines Verarbeitungsschritts an einer Probe, wobei eine Aufnahme (3) für ein eine Probe tragendes Probennahmeinstrument (4) ausgebildet ist und wobei Mittel (5) zum Ablösen der Probe von dem Probennahmeinstrument (4) ausgebildet sind, wobei die Mittel zum Ablösen ein Koppelmittel (6) zum bewegungsfesten Ankoppeln des Probenträgers (1) an einen Antrieb aufweisen, **dadurch gekennzeichnet, dass** die Aufnahme (3) für ein Einführen des Probennahmeinstruments (4) in einer durch den Probenträger (1) vorgegebenen Scheibenebene oder in einem Winkel (11) von weniger als 45° hierzu ausgerichtet ist.

9. Scheibenförmiger Probenträger (1) nach Anspruch 8, **dadurch gekennzeichnet, dass** die Mittel (5) zum Ablösen ein Koppelmittel (6) zum drehfesten Ankoppeln an einen Drehantrieb aufweisen.

10. Scheibenförmiger Probenträger (1) nach Anspruch 8 oder 9, **dadurch gekennzeichnet, dass** das Koppelmittel (6) zum drehfesten Ankoppeln an einen Drehantrieb ausgebildet ist und dass sich die Aufnahme (3) von einer nach außen offenen Einführöffnung (7) zu einem Aufnahmeende (8) erstreckt, wobei ein radialer Abstand (9) der Aufnahme (3) von einem durch das Koppelmittel (6) definierten Drehzentrum (10) längs der Aufnahme (3) zwischen der Einführöffnung (7) und dem Aufnahmeende (8) ein Minimum annimmt.

11. Scheibenförmiger Probenträger (1) nach einem der Ansprüche 8 bis 10, **dadurch gekennzeichnet, dass** die Aufnahme (3) in eine vorzugsweise in einer Strömungsrichtung (12) der abgelösten Probe hinter dem oder einem Aufnahmeende (8) der Aufnahme (3), insbesondere radial außerhalb des Aufnahmeendes (8), angeordnete Kammer (13) mündet, und/oder dass in der oder einer Strömungsrichtung (12) hinter der Aufnahme ein Filter (14) angeordnet ist.

12. Scheibenförmiger Probenträger (1) nach einem der Ansprüche 8 bis 11, **dadurch gekennzeichnet, dass** in der Aufnahme (3) ein Anlagevorsprung (15) ausgebildet ist, an welchem ein eingeführtes Probennahmeinstrument (4) punkt- oder linienförmig anliegt und/oder gehalten ist, und/oder dass ein Zytometerkanal (16) ausgebildet ist, welcher an die Aufnahme (3) angeschlossen ist.

13. Scheibenförmiger Probenträger (1) nach einem der Ansprüche 8 bis 12, **dadurch gekennzeichnet, dass** ein mit der oder einer Kammer (13) verbundenes Flüssigkeitsreservoir (17) ausgebildet und mit einer Auffangflüssigkeit befüllt ist und/oder dass ein Druckanschluss (19) für einen externen Förderdruck ausgebildet ist.

14. Scheibenförmiger Probenträger (1) nach einem der Ansprüche 8 bis 13, **dadurch gekennzeichnet, dass** das Probennahmeinstrument (4) ein saugfähiges, vorzugsweise vorbefeuchtetes, Probennahmematerial (27) aufweist und/oder dass die Kammer (13) in einer Strömungsrichtung (12) der abgelösten Probe, insbesondere radial in Bezug auf ein Drehzentrum (10) des Probenträgers (1), zulaufend ausgebildet ist und/oder an einem von der Aufnahme (3) in der oder einer Strömungsrichtung (12) der abgelösten Probe abgewandten Kammerende (20), insbesondere radial außen in Bezug auf ein Drehzentrum (10) des Probenträgers (1), einen Auslass (21) aufweist.

15. Scheibenförmiger Probenträger (1) nach einem der Ansprüche 8 bis 14, **dadurch gekennzeichnet, dass** die Aufnahme (3) eine Verlaufsrichtung hat, welche einen Winkel einschließt mit einer radialen Richtung in Bezug auf ein Drehzentrum (10) des scheibenförmigen Probenträgers (1) und/oder dass die Aufnahme (3) von einer Einführöffnung (7), insbesondere an einem äußeren Rand des scheibenförmigen Probenträgers (1), an dem oder einem Drehzentrum (10) vorbei verläuft und auf einer gegenüberliegenden Seite des Drehzentrums (10) endet.

16. Scheibenförmiger Probenträger (1) aus wenigstens zwei Segmenten (28), wobei jedes Segment (28) als scheibenförmiger Probenträger (1) nach einem der Ansprüche 8 bis 15 ausgebildet ist, insbesondere wobei die Segmente (28) jeweils paarweise entlang einer Trennlinie (29) voneinander trennbar sind und eine Einführöffnung (7) wenigstens eines Segments (28) auf der zugehörigen Trennlinie (29) liegt.

17. **Verwendung eines Probenträgers (1)** nach einem der Ansprüche 8 bis 16 bei einem Untersuchungsverfahren nach einem der Ansprüche 1 bis 7.

## Claims

1. Analysis method for a sample, wherein the sample is picked up with a sampling instrument (4) and at least one processing step is carried out on the sample on a discoid sample holder (1), wherein the sampling instrument (4) containing the picked-up sample is inserted into a slot (3) of the sample holder (1), wherein the sample is detached from the sampling instrument (4) and provided for the at least one processing step, wherein the sample is detached from the sampling instrument (4) by a movement of the sample holder (1), **characterized in that** the slot (3) for an insertion of the sampling instrument (4) is oriented in a disk plane predefined by the sample holder (1) or at an angle (11) of less than 45° to said plane.

2. Analysis method according to Claim 1, **characterized in that** the sample is detached from the sampling instrument (4) by a rotation of the sample holder (1) .

3. Analysis method according to Claim 1 or 2, **characterized in that** the detached sample is gathered in a chamber (13) before the at least one processing step, especially with an already detached sample portion being mixed in the chamber (13) with a collection liquid during the detachment.

4. Analysis method according to any of Claims 1 to 3, **characterized in that** an external conveying pressure is applied to the chamber (13) after detachment of the sample in order to convey the detached sample for the at least one processing step and/or **in that** the slot (3) is outwardly closed after insertion of the sampling instrument (4), preferably in a liquid-tight and/or gas-tight manner, especially by means of an adhesive strip and/or a stopper (18) and/or by means of a closure formed on the sampling instrument (4).

5. Analysis method according to any of Claims 1 to 4, **characterized in that** the at least one processing step is a cytometry method and/or **in that** the detached sample is filtered before entry into the chamber (13) or a chamber (13).

6. Analysis method according to any of Claims 1 to 5, **characterized in that** a swab (23) is used as sampling instrument (4), especially a swab (23) having a swab head (24) formed from tiny hairs (26) protruding from a solid core (25), and/or **in that** the sample on the sampling instrument (4) in an absorbent, preferably premoistened, sampling material (27) is inserted into the slot (3).

7. Analysis method according to any of Claims 1 to 6, **characterized in that** the sampling instrument (4) is positioned by the slot (3) at an angle to a radial direction with respect to a centre of rotation (10) of the discoid sample holder (1) and/or **in that** the sampling instrument (4) is positioned by the slot (3) from an insertion opening (7), especially at an outer edge of the discoid sample holder (1), past the centre of rotation (10) or a centre of rotation (10) and up to the slot end (8) or a slot end (8) on an opposite side of the centre of rotation (10).

8. Discoid sample holder (1) having means (2) for carrying out at least one processing step on a sample, wherein a slot (3) for a sampling instrument (4) bearing a sample is formed and wherein means (5) for the detachment of the sample from the sampling instrument (4) are formed, wherein the means for detachment comprise a coupling means (6) for the motionally fixed coupling of the sample holder (1) to a drive, **characterized in that** the slot (3) for an insertion of the sampling instrument (4) is oriented in a disk plane predefined by the sample holder (1) or at an angle (11) of less than 45° to said plane.

9. Discoid sample holder (1) according to Claim 8, **characterized in that** the means (5) for detachment comprise a coupling means (6) for rotationally fixed coupling to a rotary drive.

10. Discoid sample holder (1) according to Claim 8 or 9, **characterized in that** the coupling means (6) is formed for rotationally fixed coupling to a rotary drive and **in that** the slot (3) extends from an outwardly open insertion opening (7) to a slot end (8), with a radial distance (9) of the slot (3) from a centre of rotation (10) along the slot (3) between the insertion opening (7) and the slot end (8), which centre of rotation (10) is defined by the coupling means (6), assuming a minimum value.

11. Discoid sample holder (1) according to any of Claims 8 to 10, **characterized in that** the slot (3) opens into a chamber (13) preferably arranged after the slot end (8) or a slot end (8) of the slot (3) in a flow direction (12) of the detached sample, especially arranged radially beyond the slot end (8), and/or **in that** a filter (14) is arranged after the slot in the flow direction (12) or a flow direction (12).

12. Discoid sample holder (1) according to any of Claims 8 to 11, **characterized in that** a resting projection (15), against which an inserted sampling instrument (4) rests and/or is held in a punctiform or linear fashion, is formed in the slot (3) and/or **in that** a cytometer channel (16) connected to the slot (3) is formed.

13. Discoid sample holder (1) according to any of Claims 8 to 12, **characterized in that** a liquid reservoir (17) connected to the chamber (13) or a chamber (13) is formed and is filled with a collection liquid and/or **in that** a pressure connector (19) for an external conveying pressure is formed.

14. Discoid sample holder (1) according to any of Claims 8 to 13, **characterized in that** the sampling instrument (4) comprises an absorbent, preferably premoistened, sampling material (27) and/or **in that** the chamber (13) is formed in a tapered manner in a flow direction (12) of the detached sample, especially radially with respect to a centre of rotation (10) of the sample holder (1), and/or has an outlet (21) at a chamber end (20) facing away from the slot (3) in the flow direction (12) or a flow direction (12) of the detached sample, especially radially outward with respect to a centre of rotation (10) of the sample holder (1).

15. Discoid sample holder (1) according to any of Claims 8 to 14, **characterized in that** the slot (3) has a running direction which encloses an angle with a radial direction with respect to a centre of rotation (10) of the discoid sample holder (1) and/or **in that** the slot (3) runs from an insertion opening (7), especially on an outer edge of the discoid sample holder (1), past the centre of rotation (10) or a centre of rotation (10) and ends on an opposite side of the centre of rotation (10).

16. Discoid sample holder (1) composed of at least two segments (28), with each segment (28) being formed as a discoid sample holder (1) according to any of Claims 8 to 15, especially with the segments (28) each being separable from one another in pairs along a separation line (29) and an insertion opening (7) of at least one segment (28) lying on the associated separation line (29).

17. Use of a sample holder (1) according to any of Claims 8 to 16 in an analysis method according to any of Claims 1 to 7.

## Revendications

1. Procédé d'examen pour un échantillon dans lequel l'échantillon est prélevé avec un instrument d'échantillonnage (4) et au moins une opération de traitement est réalisée sur l'échantillon sur un porte-échantillon en forme de lame (1), dans lequel l'instrument d'échantillonnage (4) est introduit avec l'échantillon prélevé dans un réceptacle (3) du porte-échantillon (1) et dans lequel l'échantillon est détaché de l'instrument d'échantillonnage (4) et préparé pour l'au moins une opération de traitement, l'échantillon étant détaché de l'instrument d'échantillonnage (4) par un mouvement du porte-échantillon (1), **caractérisé en ce que** le réceptacle (3) pour introduire l'instrument d'échantillonnage (4) est orienté dans un plan de lame prédéfini par le porte-échantillon (1) ou selon un angle (11) de moins de 45° par rapport à celui-ci.

2. Procédé d'examen selon la revendication 1, **caractérisé en ce que** l'échantillon est détaché de l'instrument d'échantillonnage (4) par une rotation du porte-échantillon (1).

3. Procédé d'examen selon la revendication 1 ou 2, **caractérisé en ce que** l'échantillon détaché est collecté dans une chambre (13) avant l'au moins une opération de traitement, une fraction d'échantillon déjà détachée étant en particulier mélangée dans la chambre (13) avec un liquide de collecte au cours du détachement.

4. Procédé d'examen selon l'une des revendications 1 à 3, **caractérisé en ce qu'**une pression de transport externe est appliquée à la chambre (13) après le détachement de l'échantillon pour transporter l'échantillon pour l'au moins une opération de traitement et/ou que le réceptacle (3) est obturé vers l'extérieur, de préférence de façon étanche aux liquides et/ou aux gaz, après l'introduction de l'instrument d'échantillonnage (4), en particulier avec un ruban adhésif et/ou un bouchon (18) et/ou avec un moyen d'obturation formé sur l'instrument d'échantillonnage (4).

5. Procédé d'examen selon l'une des revendications 1 à 4, **caractérisé en ce que** l'au moins une opération de traitement est une procédure de cytométrie et/ou que l'échantillon détaché est filtré avant d'entrer dans la ou une chambre (13).

6. Procédé d'examen selon l'une des revendications 1 à 5, **caractérisé en ce qu'**en guise d'instrument d'échantillonnage (4) est utilisé un écouvillon (23), en particulier un écouvillon (23) qui présente une tête d'écouvillon (24) formée de poils fins (26) s'écartant d'un noyau solide (25) et/ou que l'échantillon sur l'instrument d'échantillonnage (4) est introduit dans le réceptacle (3) dans un matériau d'échantillonnage (27) absorbant, de préférence préalablement humidifié.

7. Procédé d'examen selon l'une des revendications 1 à 6, **caractérisé en ce que** l'instrument d'échantillonnage (4) est positionné à travers le réceptacle (3) sous un angle radial par rapport à un centre de rotation (10) du porte-échantillon en forme de lame (1) et/ou que l'instrument d'échantillonnage (4) est positionné à travers le réceptacle (3) depuis une ouverture d'introduction (7), en particulier au niveau d'un bord extérieur du porte-échantillon en forme de lame (1), en passant par le ou un centre de rotation (10), jusqu'à la ou une extrémité de réceptacle (8) sur un côté opposé du centre de rotation (10).

8. Porte-échantillon en forme de lame (1) avec des moyens (2) pour la réalisation d'au moins une opération de traitement sur un échantillon, dans lequel un réceptacle (3) pour un instrument d'échantillonnage (4) portant un échantillon est formé et des moyens (5) pour détacher l'échantillon de l'instrument d'échantillonnage (4) sont formés, les moyens de détachement présentant un moyen de couplage (6) pour le couplage solidaire en mouvement du porte-échantillon (1) à un dispositif d'entraînement, **caractérisé en ce que** le réceptacle (3) est orienté dans un plan de lame prédéfini par le porte-échantillon (1) ou selon un angle (11) de moins de 45° par rapport à celui-ci.

9. Porte-échantillon en forme de lame (1) selon la revendication 8, **caractérisé en ce que** les moyens de détachement (5) présentent un moyen de couplage (6) pour le couplage solidaire en rotation à un dispositif d'entraînement rotatif.

10. Porte-échantillon en forme de lame (1) selon la revendication 8 ou 9, **caractérisé en ce que** le moyen de couplage (6) est configuré pour le couplage solidaire en rotation à un dispositif d'entraînement rotatif et que le réceptacle (3) s'étend d'une ouverture d'introduction (7) ouverte vers l'extérieur à une extrémité de réceptacle (8), une distance radiale (9) du réceptacle (3) par rapport à un centre de rotation (10) défini par le moyen de couplage (6) le long du réceptacle (3) entre l'ouverture d'introduction (7) et l'extrémité de réceptacle (8) étant minimale.

11. Porte-échantillon en forme de lame (1) selon l'une des revendications 8 à 10, **caractérisé en ce que** le réceptacle (3) débouche dans une chambre (13) disposée de préférence derrière la ou une extrémité de réceptacle (8) du réceptacle (3) dans une direction d'écoulement (12) de l'échantillon détaché, en particulier radialement à l'extérieur de l'extrémité de réceptacle (8), et/ou qu'un filtre (14) est disposé derrière le réceptacle dans la ou une direction d'écoulement (12).

12. Porte-échantillon en forme de lame (1) selon l'une des revendications 8 à 11, **caractérisé en ce que** dans le réceptacle (3) est formée une saillie d'appui (15) contre laquelle un instrument d'échantillonnage (4) introduit s'appuie de façon ponctuelle ou linéaire et/ou est maintenu et/ou qu'est formé un canal de cytométrie (16) qui est raccordé au réceptacle (3).

13. Porte-échantillon en forme de lame (1) selon l'une des revendications 8 à 12, **caractérisé en ce qu'**il est formé un réservoir de liquide (17) relié avec la ou une chambre (13) qui est rempli avec un liquide de collecte et/ou qu'il est formé un raccord de pression (19) pour une pression de transport externe.

14. Porte-échantillon en forme de lame (1) selon l'une des revendications 8 à 13, **caractérisé en ce que** l'instrument d'échantillonnage (4) présente un matériau d'échantillonnage (27) absorbant, de préférence préalablement humidifié, et/ou que la chambre (13) est formée en pointe dans une direction d'écoulement (12) de l'échantillon détaché, en particulier radialement par rapport à un centre de rotation (10) du porte-échantillon (1) et/ou présente une sortie (21) à une extrémité de chambre (20) opposée au réceptacle (3) dans la ou une direction d'écoulement (12) de l'échantillon détaché, en particulier radialement à l'extérieur par rapport à un centre de rotation (10) du porte-échantillon (1).

15. Porte-échantillon en forme de lame (1) selon l'une des revendications 8 à 14, **caractérisé en ce que** le réceptacle (3) a une direction d'extension qui forme un angle avec une direction radiale par rapport à un centre de rotation (10) du porte-échantillon en forme de lame (1) et/ou que le réceptacle (3) s'étend depuis une ouverture d'introduction (7), en particulier au niveau d'un bord extérieur du porte-échantillon en forme de lame (1), en passant par le ou un centre de rotation (10), jusqu'à un côté opposé du centre de rotation (10).

16. Porte-échantillon en forme de lame (1) composé d'au moins deux segments (28) dans lequel chaque segment (28) est configuré comme un porte-échantillon à lame (1) selon l'une des revendications 8 à 15, en particulier dans lequel les segments (28) peuvent être séparés entre eux par paires le long d'une ligne de séparation (29) et dans lequel une ouverture d'introduction (7) d'au moins un segment (28) se trouve sur la ligne de séparation (29) associée.

17. Utilisation d'un porte-échantillon (1) selon l'une des revendications 8 à 16 dans un procédé d'examen selon l'une des revendications 1 à 7.
